Europäisches Patentamt

European Patent Office

Office européen des brevets

(19)

(11) Veröffentlichungsnummer: **0 001 833**
**B1**

(12) **EUROPÄISCHE PATENTSCHRIFT**

(45) Veröffentlichungstag der Patentschrift: **19.08.81**

(51) Int. Cl.³: **A 61 H 23/02 //A61N1/18**

(21) Anmeldenummer: **78101283.6**

(22) Anmeldetag: **02.11.78**

(54) Handmassagegerät.

(30) Priorität: **08.11.77 DE 2749883**

(43) Veröffentlichungstag der Anmeldung:
**16.05.79 Patentblatt 79/10**

(45) Bekanntmachung des Hinweises auf die Patenterteilung:
**19.08.81 Patentblatt 81/33**

(84) Benannte Vertragsstaaten:
**BE CH FR GB LU NL SE**

(56) Entgegenhaltungen:
**AT - B - 47 394**
**DE - A - 2 242 079**
**DE - C - 299 415**
**DE - U - 1 872 486**
**DE - U - 1 882 096**
**DE - U - 1 882 634**
**FR - A - 1 409 881**
**US - A - 1 948 067**
**US - A - 1 955 863**
**US - A - 3 710 785**

(73) Patentinhaber: **FESTO-Maschinenfabrik Gottlieb Stoll**
**Ulmer Strasse 48**
**D-7300 Esslingen (DE)**

(72) Erfinder: **Bucher, Heinz**
**Orpheusstrasse 5**
**D-7210 Rottweil (DE)**

(74) Vertreter: **Magenbauer, Rudolf, Dipl.-Ing. et al,**
**Patentanwälte, Dipl.-Ing. Rudolf Magenbauer**
**Dipl.-Phys. Dr. Otto Reimold Hölderlinweg 58**
**D-7300 Esslingen (DE)**

Courier Press, Leamington Spa, England.

Handmassagegerät

Die Erfindung betrifft ein Handmassagegerät mit einer in einem Gerätegehäuse untergebrachten elektrischen Antriebseinheit für eine in oszillierende Bewegung versetzbare Massageplatte, wobei die Massageplatte mittels eines elektrischen Heizelementes aufheizbar ist, das mit einem im Gerätegehäuse untergebrachten elektrischen Schalter verbunden ist, dessen Betätigungsorgan auf der Außenseite des Gerätegehäuses der Massageplatte abgekehrt betätigbar ist und wobei die Antriebswelle eines als Antriebseinheit verwendeten Elektromotors einen Exzenter antreibt.

Die US—A—3 710 785 zeigt bereits ein Handmassagegerät dieser Art. Bei diesem bekannten Handmassagegerät bilden die Massageplatte und das Heizelement einen großen Massagekopf, der am Gerätegehäuse abgesetzt ist. Außerdem nimmt dieser Massagekopf auch die Elemente auf, die aus der Drehbewegung der Antriebswelle des Elektromotors die oszillierende Bewegung des Massagekopfes ableiten. Bei diesem bekannten Handmassagegerät ergibt sich daher senkrecht zur Massageplatte gesehen ein hoher Aufbau, der die Handhabung des Handmassagegerätes erschwert. Darüber hinaus liegt der elektrische Anschluß des Heizelementes im Übergangsbereich zwischen dem Gerätegehäuse und dem Massagekopf frei, so daß das Handmassagegerät gerade für den Einsatz in feuchten Räumen nicht die erforderlichen Sicherheitsbestimmungen erfüllt.

Ein weiterer Nachteil liegt darin, daß das Heizelement fest mit der Rückseite der Massageplatte verbunden ist, so daß das Auswechseln der Massageplatte bzw. des Heizelementes mit Schwierigkeiten verbunden ist.

Aus der DE—U—18 82 634 ist ein Handmassagegerät bekannt, bei dem das Heizelement in einem Massage-Ansatzstück eingebettet ist, und daher nur schwer ausgewechselt werden kann. Das Massage-Ansatzstück ragt in einen offenen Kragen des Gerätegehäuses, der die Anschlüsse des Heizelementes abdeckt. Dieses Massage-Ansatzstück ist über einen Eisenbügel mit einer Magnetspule gekoppelt und wird darüber in Vibration versetzt. Auch diese starre Kopplung zwischen Massage-Ansatzstück und Magnetspule erschwert den Zugang zum Heizelement.

Es ist Aufgabe der Erfindung, ein Handgerät der eingangs erwähnten Art zu schaffen, dessen Bauhöhe senkrecht zur Massageplatte beträchtlich reduziert ist und bei dem das elektrische Heizelement so eingebaut ist, daß es alle Sicherheitsanforderungen für feuchte Räume erfüllt, aber dennoch leicht ausgetauscht werden kann.

Diese Aufgabe wird nach der Erfindung dadurch gelöst, daß die Massageplatte einen umlaufenden Rand aufweist, der in einen nach außen offenen Kragen des Gerätegehäuses ragt, daß die Massageplatte auf der Innenseite in einem hülsenförmigen Ansatz den in einem Exzentergehäuse gelagerten Exzenter aufnimmt, daß die Massageplatte mittels bekannter elastischer Schwingelemente an dem Elektromotor befestigt ist und daß die Massageplatte innerhalb des Randes einen Durchbruch für die Anschlußleitung zu einem plattenförmigen Heizelement aufweist, das in einer rückseitigen Vertiefung einer Deckplatte angeordnet ist, die auf die Massageplatte aufgerastet ist.

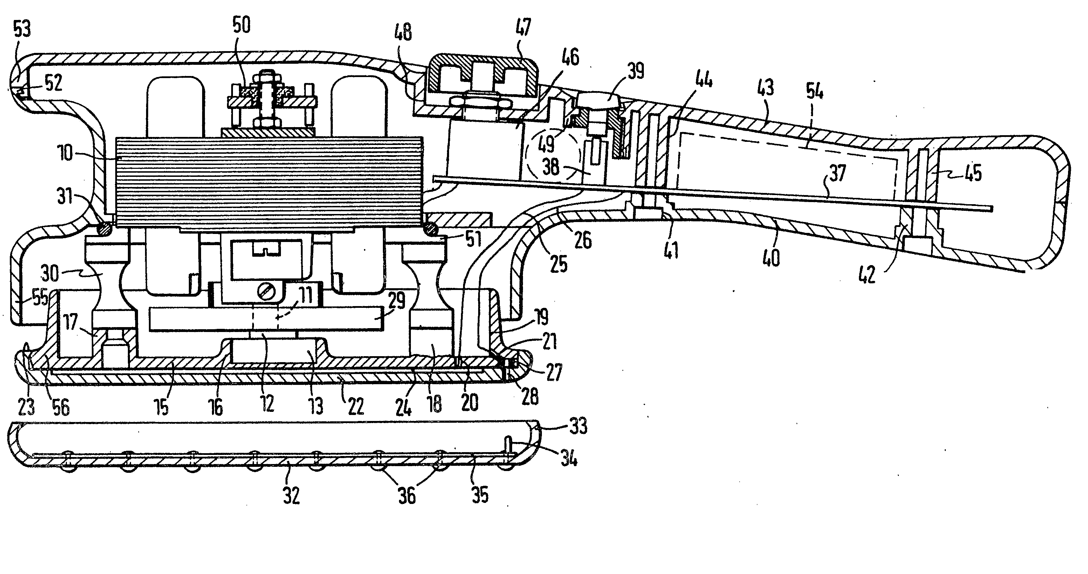

Durch diese Ineinanderschachtelung von Massageplatte und Gerätegehäuse wird ein stets vollständig abgedeckter Kanal zur Führung der Anschlußleitung für das Heizelement geschaffen. Mit Hilfe der zusätzlichen Deckplatte wird das Heizelement so abgedeckt, daß es für eine Reparatur zugänglich bleibt. Außerdem übernimmt jetzt die Deckplatte die Funktion der Massageplatte, wobei durch unterschiedliche Ausgestaltung der Außenseite der Deckplatte unterschiedliche Massagewirkung erzielt werden kann. Der direkte Antrieb der Massageplatte durch den vom Elektromotor angetriebenen Exzenter und die Aufhängung der Massageplatte über an sich bekannte elastische Schwingelemente am Elektromotor bringen die minimale Bauhöhe für das Handmassagegerät, wie der Schnitt nach der Zeichnung deutlich erkennen läßt.

Der Zugang zum Heizelement kann dadurch ohne Trennung der Wirkverbindung zwischen Antriebseinheit und Massageplatte erreicht werden, daß die Deckplatte mit einem umlaufenden Rastrand versehen ist, der auf einen umlaufenden Rastabsatz der Massageplatte aufrastbar ist.

Das Handmassagegerät kann nach einer Weiterbildung dadurch als Reizstromgerät mit ausgenützt werden, daß die Massageplatte mit zwei Steckanschlüssen versehen ist, daß die Massageplatte mit einer Elektrodenplatte verbindbar ist, die mit entsprechenden Steckelementen versehen ist, daß die Steckanschlüsse der Massageplatte mittels einer Verbindungsleitung mit einem Reizstromgenerator verbunden sind, der auf der Leiterplatte angeordnet ist, daß die die Massageplatte abdeckende Deckplatte im Bereich der Steckanschlüsse mit Durchbrüchen versehen ist, daß der Reizstromgenerator mit einem Einstellglied versehen ist und daß dieses Einstellglied mittels eines Schaltgliedes verstellbar ist, das in einer vertieften Aufnahme des Deckels angeordnet und auf der Außenseite desselben zugänglich ist.

Die Ausgestaltung ist dabei zweckmäßigerweise so, daß die Elektrodenplatte zwei Gruppen von Elektroden aufweist, die über Leiterbahnen mit den beiden Steckelementen der Elektrodenplatte verbunden sind und daß die Elektrodenplatte mittels eines Rastrandes auf die Deckplatte lösbar aufrastbar ist.

Die Erfindung wird anhand eines in der Zeichnung im Längsschnitt dargestellten Ausführungsbeispiels näher erläutert.

Die electrische Antriebseinheit des Handmassagegerätes umfaßt einen Elektromotor 10, der mittels seines Lagerschildes 51 und einer Dämpfungsmanschette 50 in dem Gehäusegrundteil 40 des Gerätegehäuses elastisch und geräuschgedämpft aufgehängt ist. Dabei wird der Lagerschild 51 über einen Dichtungsring gegen Stege des Gehäusegrundteils 40 gedrückt.

Die Motorwelle 11 trägt ein Lüfterrad 29 und einen Exzenter 12, der in dem Exzentergehäuse 13 drehbar ist. Das Exzentergehäuse 13 ist in einem hülsenförmigen Ansatz 16 auf der Rückseite der Massageplatte 15 festgelegt. Außerdem weist die Massageplatte 15 Befestigungshülsen 17 und 18 auf, an denen an sich bekannte Schwingelemente 30 aus Gummi befestigt sind. Diese Schwingelemente 30 sind an dem Lagerschild 51 des Electromotors 10 angebracht und erlauben die oszillierende Bewegung der Massageplatte 15, wenn die Motorwelle 11 den Exzenter 12 in Drehbewegungen versetzt. Die Massageplatte 15 ragt dabei mit ihrem umlaufenden Rand 19 frei in den nach außen offenen Kragen 55 des Gehäusegrundteiles 40.

Die Massageplatte 15 weist einen umlaufenden Rastabsatz 56 auf, auf den eine Deckplatte 22 mit umlaufendem Rastrand 23 unlösbar aufrastbar ist. Die Deckplatte 22 weist auf der Innenseite eine Vertiefung auf, in die ein plattenförmiges Heizelement 24 eingelegt ist. Die Anschlußleitung 25 zu dem Heizelement 24 ist dabei durch den Durchbruch 20 der Massageplatte 15 geführt und mit einem elektrischen Schalter 38 verbunden. Das Heizelement 24 ist voll abgedeckt und der Anschluß ist so in das Innere des Gerätegehäuses geführt, daß eine Berührung mit stromführenden Teilen sicher verhindert ist. Das Heizelement kann daher wie der Elektromotor 10 direkt von der Netzwechselspannung gespeist werden.

Der elektrische Schalter 38 ist auf einer Leiterplatte 37 befestigt, die beim Zusammenbau des Gehäusegrundteiles 40 und des Deckels 43 festgelegt wird. Das Gehäusegrundteil 40 und der Deckel 43 bilden mit Ansätzen einen hohlen Handgriff, der die Leiterplatte 37 mit dem elektrischen Schalter 38 aufnimmt. Das Gehäusegrundteil 40 weist Befestigungshülsen 41 und 42 auf, während am Deckel 43 entsprechend verteilt Schraubstutzen 44 und 45 angeformt sind. Die Leiterplatte 37 weist darauf abgestimmte Bohrungen auf und wird mittels der Befestigungsschrauben zwischen den Befestigungshülsen 41 und 42 sowie den Schraubstutzen 44 und 45 gehalten.

In einer vertieften Aufnahme 49 des Deckels 43 ist ein getrenntes Betätigungsorgan 39 unverlierbar gehalten, welches mit dem mechanischen Auslöseglied des als Mikroschalter ausgebildeten elektrischen Schalters 38 in Wirkverbindung steht. Der elektrische Schalter 38 kann daher von außen geschaltet werden. Das Betätigungsorgan 39 ist von den Kontakten des elektrischen Schalters 38 eindeutig isoliert.

Der Deckel 43 läuft außerhalb des Handgriffes in einen Nut-Feder-Rand 53 auf, der in den Nut-Feder-Gegenrand 52 des Gehäusegrundteiles 40 eingeführt werden kann. Auf diese Weise werden der Deckel 43 und das Gehäusegrundteil 40 auch im Aufnahmebereich für die elektrische Antriebseinheit dicht und eindeutig miteinander verbunden.

Ferner sind in der Massageplatte 15 zwei Steckanschlüsse 27 festgelegt, die über Bohrungen 28 der Deckplatte 22 erreichbar sind. Diese Steckanschlüsse 27 dienen zur elektrischen Durchschaltung, wenn die Elektrodenplatte 32 auf die Deckplatte 22 aufgerastet wird. Dabei werden die Steckelemente 34 in die Steckanschlüsse 27 eingeführt. Die Elektrodenplatte 32 weist auf der Außenseite eine Vielzahl von Elektroden 36 auf, die über Leiterbahnen 35 auf der Innenseite in zwei Gruppen zusammengefaßt sind, welche mit den beiden Steckelementen 34 in Verbindung stehen.

Die zweiadrige Verbindungsleitung 26 ist durch die Durchbrüche 21 der Massageplatte 15 geführt und mit den beiden Steckanschlüssen 27 verbunden. Außerdem ist diese Verbindungsleitung 26 auf der Leiterplatte 37 mit dem Ausgang eines Reizstromgenerators 54 verbunden, der schwachen Gleichstrom, Gleichstrom- oder Wechselstromimpulse, oder auch hochfrequenten Reizstrom abgeben kann. Auf der Leiterplatte 37 ist ein Einstellglied 46 angeordnet, das z.B. als Drehwiderstand mit einem Schaltkontakt ausgebildet sein kann. Das Schaltglied 47 des Einstellgliedes 46 ist in der vertieften Aufnahme 48 des Deckels 43 angeordnet. Durch Druck auf das Schaltglied 47 kann der Schaltkontakt gesteuert und so der Reizstromgenerator 54 ein- und ausgeschaltet werden. Durch Drehen des Schaltgliedes 47 kann der Drehwiderstand verändert und so die Amplitude, die Frequenz- oder eine andere Betriebsgröße zur Beeinflussung der Reizstrom-Massagewirkung eingestellt werden.

Die Elektroden 36 der Elektrodenplatte 32 bilden eine Vielzahl von Strompfaden, denen der Reizstrom über die Verbindungsleitung 26 zugeführt werden kann.

Die Elektrodenplatte 36 kann von der Deckplatte 22 wieder gelöst werden, wobei die elektrische Durchschaltung zum Ausgang des Reizstromgenerators 54 selbsttätig unterbrochen wird.

Mit dem neuen Handmassagegerät kann daher die mechanische Massage wahlweise nicht nur mit einer Wärmeeinwirkung, sondern auch mit einer variablen Reizstrommassage kombiniert werden.

Die Wärmeeinwirkung kann dadurch in einfacher Weise reguliert werden, daß der elektrische Schalter mit einem Einstellwiderstand zur Einstellung des Heizstromes für das Heizelement ausgerüstet ist, welcher mittels des Schalterbetätigungsorganes veränderbar ist. Dieser Einstellwiderstand ist als Regelwiderstand dem Heizelement einfach vorgeschaltet.

Aus Sicherheitsgründen ist zusätzlich vorgesehen, daß auf der Leiterplatte bzw. im Bereich des Heizelementes ein Siche heitsthermostat zur Abschaltung des Heizkreises beim Überschreiten einer vorgegebenen maximalen Temperatur angeordnet ist. Die Massageplatte kann daher nicht zu stark erwärmt erden.

**Patentansprüche**

1. Handmassagegerät mit einer in einem Gerätegehäuse untergebrachten elektrischen Antriebseinheit für eine in oszillierende Bewegung versetzbare Massageplatte (15), wobei die Massageplatte (15) mittels eines elektrischen Heizelementes (24) aufheizbar ist, das mit einem im Gerätegehäuse (40, 43) untergebrachten elektrischen Schalter (3) verbunden ist, dessen Betätigungsorgan (39) auf der Außenseite des Gerätegehäuses (40, 43) der Massageplatte (15) abgekehrt betätigbar ist und wobei die Antriebswelle (11) eines als Antriebseinheit verwendeten Elektromotors (10) einen Exzenter (12) antreibt, dadurch gekennzeichnet, daß die Massageplatte (15) einen umlaufenden Rand (19) aufweist, der in einen nach außen offenen Kragen (55) des Gerätegehäuses (40, 43) ragt, daß die Massageplatte (15) auf der Innenseite in einem hülsenförmigen Ansatz (16) den in einem Exzentergehäuse (13) gelagerten Exzenter (12) aufnimmt, daß die Massageplatte (15) mittels bekannter elastischer Schwingelemente (30) an dem Elektromotor (10) befestigt ist und daß die Massageplatte (15) innerhalb des Randes (19) einen Durchbruch (20) für die Anschlußleitung (25) zu einem plattenförmigen Heizelement (24) aufweist, das in einer rückseitigen Vertiefung einer Deckplatte (22) angeordnet ist, die auf die Massageplatte (15) aufgerastet ist.

2. Handmassagegerät nach Anspruch 1, dadurch gekennzeichnet, daß die Deckplatte (22) mit einem umlaufenden Rastrand (23) versehen ist, der auf einen umlaufenden Rastabsatz (56) der Massageplatte (15) aufrastbar ist.

3. Handmassagegerät nach Anspruch 1 und 2, dadurch gekennzeichnet, daß die Massageplatte (15) mit zwei Steckanschlüssen (27) versehen ist, daß die Massageplatte (15) mit einer Elektrodenplatte (32) verbindbar ist, die mit entsprechenden Steckelementen (34) versehen ist, daß die Steckanschlüsse (27) der Massageplatte (15) mittels einer Verbindungsleitung (26) mit einem Reizstromgenerator (54) verbunden sind, der auf der Leiterplatte (37) angeordnet ist, daß die die Massageplatte (15) abdeckende Deckplatte (22) im Bereich der Steckanschlüsse (27) mit Durchbrüchen (28) versehen ist, daß der Reizstromgenerator (54) mit einem Einstellglied (46) versehen ist und daß dieses Einstellglied (46) mittels eines Schaltgliedes (47) verstellbar ist, das in einer vertieften Aufnahme (48) des Deckels (43) angeordnet und auf der Außenseite desselben zugänglich ist.

4. Handmassagegerät nach Anspruch 3, dadurch gekennzeichnet, daß die Elektrodenplatte (32) zwei Gruppen von Elektroden (36) aufweist, die über Leiterbahnen (35) mit den beiden Steckelementen (34) der Elektrodenplatte (32) verbunden sind und daß die Elektrodenplatte (32) mittels eines Rastrandes (33) auf die Deckplatte (22) lösbar aufrastbar ist.

**Revendications**

1. Appareil de massage à main qui comporte, logée dans un boîtier, une unité d'entraînement électrique d'une plaque de massage vibratoire qui peut être chauffée par un élément chauffant électrique connecté à un interrupteur logé dans ledit boîtier et dont l'organe de manoeuvre, situé à l'opposé de ladite plaque de massage, peut être actionné sur la face externe dudit boîtier, appareil dans lequel l'arbre menant d'un moteur électrique, utilisé comme unité d'entraînement, entraîne un excentrique, appareil caractérisé en ce que la plaque de massage (15) présente un rebord périphérique (19) qui pénètre dans un collet (55) du boîtier (40, 43), ouvert vers l'extérieur; en ce que, dans une saillie (16) en forme de douille ménagée sur sa face interne, ladite plaque de massage (15) loge un excentrique (12) situé dans une enveloppe (13) et entraîné par l'arbre menant (11) d'un moteur électrique (10); en ce que ladite plaque de massage (15) est fixée au moteur d'entraînement (10) par l'intermédiaire de tampons oscillants élastiques connus (30); et en ce que ladite plaque de massage (15) comporte, dans son rebord (19), une ouverture (20) assurant le passage du conducteur de raccordement (25) d'un élément chauffant électrique (24) en forme de plaque, disposé dans un évidement ménagé dans la face postérieure d'une plaque de couverture (22) encliquetée sur ladite plaque de massage (15).

2. Appareil selon la revendication 1, caractérisé en ce que la plaque de couverture (22) présente un rebord périphérique encliquetable (23) dans lequel peut être emboîtée une saillie périphérique encliquetable (56) de la plaque de massage (15).

3. Appareil selon l'une des revendications 1 et 2, caractérisé en ce que la plaque de massage (15), comportant deux prises de raccordement (27), peut être connectée à une plaque (32) à électrodes présentant des fiches ou tétons complémentaires (34); en ce que lesdites prises de raccordement (27) de ladite plaque de massage (15) sont connectées, au moyen d'un conducteur de raccordement (26), à un générateur (54) de courant d'excitation monté sur la plaquette conductrice (37); en ce que la plaque de couverture (22) de ladite plaque de massage (15) est percée d'orifices (28) dans la région desdites prises de raccordement (27); en ce que ledit générateur (54) est équipé d'un organe de réglage (46); et en ce que ledit organe (46) est réglable au moyen d'un organe de commutation (47) logé dans un évidement profond (48) du couvercle (43) et accessible sur la face externe de ce dernier.

4. Appareil selon la revendication 3, caractérisé en ce que la plaque (32) à électrodes présente deux groupes d'électrodes (36) qui sont raccordés par des pistes conductrices (35) aux deux tétons enfichables (34) de ladite plaque (32), et en ce que cette dernière peut être emboîtée de manière amovible sur la plaque de couverture (22) au moyen d'un bord encliquetable (33).

## Claims

1. Hand-held massager including a drive unit accommodated in an appliance housing, for driving a massaging head which is caused to perform vibratory motion, with said massaging head capable of being heated with the aid of an electric heating element which is connected to an electric switch accommodated in the housing of the massager, with the actuating member thereof capable of being actuated on that outer side of the housing not facing said massaging head, characterized in that said massaging head (15) is provided with a peripheral rim portion (19) protruding into an outwardly open collar (55) of said appliance housing (40, 43), that said massaging head (15) on its inside, in a sleeve-shaped extension (16), contains a housing of an eccenter (12) driven by the drive shaft (11) of an electric motor (10), that said massaging head (15) is secured to said electric motor (10) with the aid of conventional types of flexible swing elements (30), and that said massaging head (15), within said rim portion (19), is provided with a grommet (20) for the connecting cable (25) extending to a plate-shaped heating element (24) as arranged in a recess on the rear side of a cover plate (22) which is snapped onto said massaging head (15).

2. A hand-held massager as claimed in claim 1, characterized in that said cover plate (22) comprises a peripheral snap-lock rim portion (23) which is capable of snapping onto a peripheral snap projection (56) provided for on said massaging head (15).

3. A hand-held massager as claimed in claims 1 and 2, characterized in that said massaging head (15) is provided with two socket terminals (27), that said massaging head (15) is capable of being connected to an electrode plate (32) which is provided with the corresponding pin terminals (34), that said socket terminals (27) of said massaging head (15), via a lead-in wire (26), are in connection with an exciting-current generator (54) which is arranged on the printed circuit board (37), that said cover plate (22) covering said massaging head (15), is provided with openings (28) within the range of said socket terminals (27), that said exciting-current generator (54) is provided with an adjusting element (46), and that said adjusting element (46) is capable of being adjusted with the aid of a switch member (47) arranged in a recessed receptacle (48) provided for in said cover (43) and is accessible from the top side of said cover.

4. A hand-held massager as claimed in claim 3, characterized in that said electrode plate (32) comprises two groups of electrodes (36) which, via conductor leads (35), are in connection with said two pin terminals (34) of said electrode plate (32), and that said electrode plate (32), by means of a snap-lock rim portion (33), is capable of snapping releasably onto said cover plate (22).

0 001 833

1